# FASCICULE DE BREVET EUROPEEN

(11) **EP 2 787 895 B1**
(45) Date de publication et mention de la délivrance du brevet: **29.05.2019**
(21) Numéro de dépôt: 12795486.5
(22) Date de dépôt: 06.12.2012
(51) Int. Cl.: A61B 34/30, A61B 90/50, A61B 34/00, A61B 34/20

(54) **DISPOSITIF D'ASSISTANCE AU POSITIONNEMENT D'UN INSTRUMENT MEDICAL RELATIVEMENT A UN ORGANE INTERNE D'UN PATIENT**
UNTERSTÜTZUNGSVORRICHTUNG ZUR POSITIONIERUNG EINES MEDIZINISCHEN INSTRUMENTES IM VERHÄLTNIS ZU EINEM INNEREN ORGAN EINES PATIENTEN
ASSISTIVE DEVICE FOR POSITIONING A MEDICAL INSTRUMENT RELATIVE TO AN INTERNAL ORGAN OF A PATIENT

(30) Priorité: 06.12.2011 FR 1161217
(43) Date de publication de la demande: 15.10.2014
(73) Titulaire: Sorbonne Université, 75006 Paris (FR); Koelis, 38240 Meylan (FR); Centre National de la Recherche Scientifique, 75016 Paris (FR)
(72) Inventeur: POQUET, Cécile, F-94200 Ivry-Sur-Seine (FR); MOZER, Pierre, F-94300 Vincennes (FR); BAUMANN, Michael, F-38100 Grenoble (FR); VITRANI, Marie-Aude, F-95013 Paris (FR); MOREL, Guillaume, F-95013 Paris (FR); LEROY, Antoine, 78390 Bois d'Arcy (FR); HENRI, Patrick, décédé (FR); COFFIN, Grégoire, F-75015 Paris (FR)
(74) Mandataire: Decorchemont, Audrey Véronique Christèle
(86) Numéro de dépôt international: PCT/EP2012/074711
(87) Numéro de publication internationale: WO 2013/083731

(56) Documents cités:
- WO-A1-2006/091494
- US-A1- 2003 097 060
- US-A1- 2006 207 978
- US-A1- 2009 118 640
- US-A1- 2010 056 900
- GREMINGER M A ET AL: "Vision-based force measurement", IEEE TRANSACTIONS ON PATTERN ANALYSIS AND MACHINE INTELLIGENCE, IEEE SERVICE CENTER, LOS ALAMITOS, CA, US, vol. 26, no. 3, 1 mars 2004 (2004-03-01), pages 290-298, XP011106112, ISSN: 0162-8828, DOI: 10.1109/TPAMI.2004.1262305

## Description

L'invention concerne un dispositif d'assistance au positionnement d'un instrument médical relativement à un organe interne d'un patient. L'invention concerne également un procédé de commande d'un tel dispositif.

De façon plus précise, l'invention concerne un dispositif d'assistance au positionnement d'un instrument médical relativement à un organe interne d'un patient qui comporte un organe de commande de moyens de déplacement dudit instrument médical, l'organe de commande étant relié à des moyens d'acquisition d'images de l'organe interne et comportant des moyens d'analyse d'images pour générer des ordres de commande à destination des moyens de déplacement.

### ARRIERE PLAN TECHNOLOGIQUE DE L'INVENTION

Les opérations dites « ouvertes » s'avèrent très lourdes pour un patient. Ainsi, de plus en plus, les praticiens ont recours à des opérations mini-invasives lors desquelles des instruments médicaux sont insérés dans une voie naturelle du patient (vagin, rectum, conduit auditif...) ou dans une voie artificielle rapportée sur le corps du patient (canule, veine artificielle, trocart...).

En urologie, afin de dépister un éventuel cancer de la prostate, il est connu de réaliser une biopsie prostatique. Il s'agit de prélever des échantillons de tissus au sein même de la prostate, lesdits échantillons étant ensuite analysés en laboratoire afin de détecter la présence d'éventuelles cellules cancéreuses. A cet effet, le patient est couché sur le flanc. Un instrument médical, comportant une sonde échographique et un porte-aiguille portant une aiguille à biopsie, est alors inséré dans la voie naturelle qu'est le rectum. Grâce à l'instrument médical, le chirurgien perce la paroi du colon pour atteindre la prostate et prélever ainsi des échantillons de tissus.

Pour réaliser la biopsie, le chirurgien peut seulement s'aider des images en deux dimensions prises en temps réel par la sonde échographique. Le chirurgien doit ainsi estimer de tête une représentation en trois dimensions de la prostate pour réaliser les prélèvements d'échantillons distribués régulièrement dans le volume de la prostate. Ceci nécessite donc une très grande dextérité de la part du chirurgien.

Par ailleurs, la prostate est un organe extrêmement mou de sorte que même le simple contact de l'aiguille, sans que celle-ci soit enfoncée dans la prostate, suffit à déformer ladite prostate. Ainsi, même si le chirurgien pense avoir réalisé correctement les prélèvements, il est possible qu'il n'ait pas piqué exactement là ou il le fallait. Les prélèvements risquent alors de ne pas être réalisés de façon homogène sur la prostate.

Récemment, des dispositifs sont apparus afin d'aider le chirurgien à réaliser très précisément les différentes ponctions.

Des nouveaux dispositifs comportent des sondes permettant de fournir des images en trois dimensions de la prostate. Le principe de fonctionnement desdits plannings est de réaliser un planning de positions de ponctions théoriques et d'étudier au cours de la biopsie la déformation de la prostate. Le planning de ponctions théoriques est alors modifié en fonction de l'étude de la déformé de la prostate afin de réaliser les ponctions aux bons endroits. Toutefois, le placement correct des ponctions selon le nouveau planning reste entièrement dépendant de l'habilité du chirurgien.

En plus de cela, il se peut que le patient ne soit pas sous anesthésie locale et/ou qu'il ne soit pas immobilisé. Ainsi, lors de l'intervention, le patient peut bouger et avec lui tous les tissus de la voie naturelle ou artificielle avec lesquels l'instrument médical peut entrer en contact.

Afin d'aider le chirurgien, il est connu des dispositifs de guidage d'instrument médical comportant un bras articulé pour déplacer une extrémité proximale de l'instrument médical. Le chirurgien peut alors commander le bras articulé pour que celui-ci bloque l'instrument médical dans une position donnée. Toutefois, de tels dispositifs de guidage considèrent des positions de l'instrument médical définies dans un référentiel fixe par rapport à la salle d'examen dans laquelle est placé le patient.

Or le patient et/ou sa prostate peuvent bouger ce qui a deux implications majeures.

Tout d'abord, la prostate n'est donc pas fixe par rapport au référentiel dans lequel est définie la commande de position du bras articulé. Il se peut donc que la prostate ait changé de position entre le moment où la position désirée de l'instrument médical est donnée au bras articulé et le moment où cette position est atteinte par l'instrument médical. Ainsi, la cible atteinte par l'instrument médical peut être correcte du point de vue du bras articulé dans le référentiel fixe mais erronée du point de vue de la prostate : la ponction n'est pas réalisée dans la zone prévue de la prostate.

Ensuite, le point d'entrée de l'instrument médical dans le corps du patient bouge avec le patient. Ainsi, le bras articulé peut s'opposer aux mouvements du patient, ce qui peut incommoder le patient voir endommager une partie de ses tissus.

Lors de l'intervention, le point d'entrée de l'instrument médical dans le patient génère sur l'instrument médical des forces « parasites », qui noient l'information tactile utile au chirurgien, à savoir les efforts d'interaction entre l'instrument médical et l'organe ciblé. De ce fait, le chirurgien perçoit mal les efforts appliqués sur la prostate et peut produire des grandes déformations et/ou des grands déplacements de la prostate sans en avoir conscience.

US 2010/056900, WO 2006/091494 et US 2003/097060 sont des documents pertinents pour l'invention.

### OBJET DE L'INVENTION

Un but de l'invention est de proposer un dispositif d'assistance au positionnement d'un instrument médical inséré dans une voie naturelle ou une voie artificielle d'un patient relativement à un organe interne qui obvie au moins en partie aux inconvénients précités.

### BREVE DESCRIPTION DE L'INVENTION

L'invention est definie par la revendication indépendante. En vue de la réalisation de ce but, on propose un dispositif d'assistance au positionnement d'un instrument médical inséré dans une voie naturelle ou une voie artificielle d'un patient relativement à un organe interne d'un patient, le dispositif comportant :
- un support destiné à être inséré au moins en partie dans le corps du patient et qui porte l'instrument médical ;
- des moyens de déplacement du support, lesdits moyens de déplacement comportant un bras articulé qui comprend une pluralité de degrés de liberté pour déplacer une extrémité proximale du support ;
- des moyens d'acquisition d'images de l'organe interne pour positionner l'instrument médical relativement à l'organe interne, les moyens d'acquisition d'images comportant une sonde portée par le support de sorte que l'instrument médical et la sonde soient rigidement liés ;
- un organe de commande des moyens de déplacement qui est relié aux moyens d'acquisition d'images et qui comporte des moyens d'analyse des images pour générer des ordres de commande à destination du bras articulé afin de contrôler au moins un déplacement de l'instrument médical relativement à l'organe interne, les moyens d'analyse comportant des moyens d'estimation d'une distance entre la sonde et l'organe interne pour freiner un déplacement du bras articulé en fonction de ladite distance de façon que le bras articulé (9) soit de plus en plus freiné au fur et à mesure que l'instrument (2) s'approche du organe, les moyens d'analyse comportant des moyens d'estimation d'une déformée de l'organe interne pour freiner un déplacement du bras articulé en fonction de ladite déformée de façon que le bras articulé (9) soit de plus en plus freiné au fur et à mesure que l'instrument (2) s'enfonce dans l'organe.

Ainsi, le support porte à la fois la sonde et l'instrument médical de sorte que le positionnement relatif de la sonde par rapport à l'instrument médical est connu. Les images prises par la sonde sont représentatives d'une position de la sonde vis-à-vis de l'organe interne et donc également directement représentatives de la position de l'instrument médical vis-à-vis de l'organe interne. Dès lors, il est possible d'estimer en temps réel la position de l'instrument médical vis-à-vis de l'organe interne, sans recalage.

Le dispositif comporte de plus un bras articulé comportant une pluralité de degrés de liberté pour déplacer l'extrémité proximale du support en générant des efforts en cette extrémité. Du fait de l'insertion de l'instrument médical dans le patient, la position de l'instrument médical est bien définie et peut être modifiée par le bras articulé pouvant agir sur la position de l'extrémité proximale du support et par là de l'instrument médical.

Le bras articulé n'est pas le seul à agir sur la position de l'instrument médical: un chirurgien manipule également le support et donc l'instrument médical. Ainsi, c'est la combinaison des efforts exercées sur le support par le chirurgien d'une part et par le bras articulé d'autre part qui va déterminer les mouvements et la position de l'instrument médical.

Ainsi le support est manipulé à la fois par le chirurgien et par le bras articulé, ce qui permet d'agir sur la position de l'instrument médical et sur le ressenti tactile du chirurgien.

En effet, la génération d'efforts par le bras articulé n'a pas pour but de manipuler seul l'instrument médical, mais de modifier le ressenti du chirurgien et donc ses gestes, notamment par exemple en lui permettant de ressentir des efforts représentatifs de l'interaction entre l'instrument médical et l'organe ou en modifiant son geste de façon à positionner l'instrument médical dans la position cible.

Par ailleurs, grâce aux moyens d'estimation de la distance entre la sonde et l'organe interne, l'organe de commande génère des ordres de commande pour opposer une résistance au déplacement de l'instrument médical en fonction de ladite distance de façon que le support soit de plus en plus freiné au fur et à mesure que l'aiguille s'approche de la prostate.

Encore une fois, le dispositif de l'invention permet d'assister le chirurgien dans le geste qu'il a à accomplir sur l'organe interne ce qui évite par exemple que le chirurgien appuie trop fortement ou trop rapidement avec l'instrument médical sur l'organe interne.

Le dispositif de l'invention est particulièrement intéressant pour approcher un instrument médical d'un élément mou comme la prostate dont les déformations sont extrêmement fréquentes.

En effet, grâce aux images de la sonde, il devient possible de prendre en compte la déformation de la prostate pour en positionner correctement l'instrument médical vis-à-vis de la prostate malgré la déformation de cette dernière. Il est donc possible de réaliser une biopsie à un endroit très précis.

En outre, avec les dispositifs de l'art antérieur, il a été constaté qu'à cause notamment de la grande déformabilité de la prostate, la plupart des chirurgiens estimaient parfois ne pas encore avoir atteint la prostate P alors qu'ils la comprimaient déjà. Grâce au dispositif de l'invention, le chirurgien peut être guidé jusqu'à la prostate de sorte à venir reposer l'instrument médical sur elle sans la comprimer ce qui permet de bien mieux placer l'instrument médical relativement à la prostate avant de réaliser un geste médical sur ladite prostate.

### BREVE DESCRIPTION DES DESSINS

L'invention sera mieux comprise à la lumière de la description qui suit d'un mode de réalisation particulier non limitatif de l'invention en référence à la figure unique qui est une vue schématique d'un dispositif d'assistance de l'invention, une partie du corps d'un patient étant représentée.

### DESCRIPTION DETAILLEE DE L'INVENTION

Le dispositif d'assistance au positionnement d'un instrument médical relativement à un organe interne d'un patient selon l'invention est ici destiné à approcher un instrument médical à proximité d'une prostate. Cette application n'est bien sûr pas limitative et on pourra approcher, grâce au dispositif de l'invention, un instrument médical à proximité de n'importe quel organe interne, par exemple une vessie.

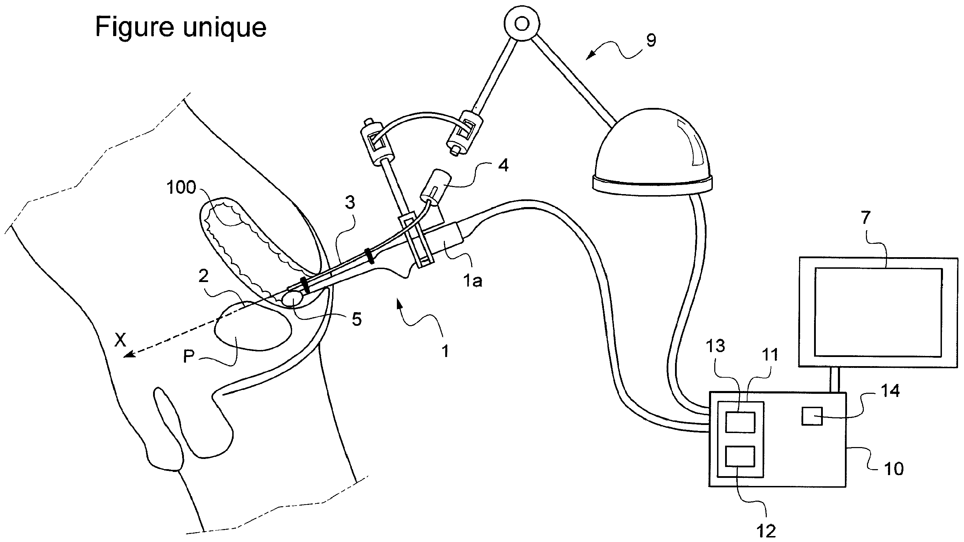

En référence à la figure 1, le dispositif comporte un support 1 comportant un porte-aiguille 3 qui reçoit une aiguille 2 pour la réalisation d'une biopsie sur une prostate P d'un patient. Le support 1 comporte en outre des moyens d'actionnement 4 du porte-aiguille 3 pour insérer et retirer l'aiguille 2 de la prostate P selon un axe de travail X déterminé.

Le support 1 est destiné à être introduit, au moins en partie, dans le corps du patient pour que l'aiguille 2 soit approchée de la prostate P. A cet effet, le dispositif comporte des moyens de déplacement du support 1. Lesdits moyens comportent un bras articulé 9 qui comprend une pluralité de degrés de liberté pour déplacer une extrémité proximale la du support. Par extrémité proximale, on entend l'extrémité opposée à celle introduite dans le corps du patient.

Le dispositif comporte également des moyens d'acquisition d'images de la prostate P pour positionner l'aiguille 2 relativement à la prostate P. Les moyens d'acquisition d'images comportent une sonde échographique 5 par exemple une sonde échographique ultrasonore. La sonde 5 est, selon l'invention, portée par le support 1 de sorte que la sonde 5 et le porte-aiguille 3 et l'aiguille 2 soient rigidement liés et introduits ensembles dans le rectum 100.

Ici, le support 1 est introduit par la voie naturelle qu'est le rectum 100 en passant par l'anus jusqu'à faire venir la sonde 5 au contact de la paroi rectale en regard de la prostate P, et l'aiguille 2 est insérée lors de la biopsie dans la prostate P à travers ladite paroi rectale.

Ainsi, des images prises par la sonde 5 sont représentatives de la position de support 1 vis-à-vis de la prostate P. Comme la sonde 5 est à une distance déterminée de l'aiguille 2, dont le porte-aiguille 3 est également rigidement lié au support 1, ces images sont aussi représentatives d'une position de l'aiguille 2 vis-à-vis de la prostate P. Il est donc possible de déterminer en temps réel, la position de l'aiguille 2 vis-à-vis de la prostate P ce qui facilite grandement le travail d'un praticien. Il convient de noter que la paroi rectale est très fine de sorte qu'elle ne gêne pas l'acquisition d'images de la prostate P par la sonde 5.

Le dispositif de l'invention comporte en outre un organe de commande 10 des moyens de déplacement, et notamment du bras articulé 9, qui est relié aux moyens d'acquisition d'images, et notamment de la sonde 5. L'organe de commande 10 comporte des moyens d'analyse 11 desdites images pour générer des ordres de commande à destination du bras articulé 9 afin de contrôler au moins un déplacement de l'aiguille 2 relativement à la prostate P.

L'organe de commande 10 positionne donc l'aiguille 2 relativement à la prostate P par l'intermédiaire du bras articulé 9 grâce aux images de la sonde 5. En outre, puisqu'aucun recalage n'est nécessaire, l'organe de commande 10 peut ajuster en temps réel les ordres de commande pour positionner correctement l'aiguille 2 vis-à-vis de la prostate P lorsque cette dernière s'est déformée ou s'est déplacée.

Ainsi, lorsque le support 1 et les moyens de déplacement sont bloqués et que l'aiguille 2 repose contre la prostate P, les moyens d'analyse 11 peuvent déduire des images de la prostate P si la prostate P a bougé relativement à la sonde 5. Si tel est le cas, il est dès lors nécessaire de réajuster la position de l'aiguille 2 vis-à-vis de la prostate P. L'organe de commande 10 assure ainsi en permanence un placement précis de l'aiguille 2 vis-à-vis de la prostate P.

Les inventeurs se sont aperçus que certains praticiens avaient tendance à trop presser le support 1 contre la prostate P. En effet à cause d'un manque de contact visuel et de la grande déformabilité de la prostate, les praticiens estiment parfois ne pas encore avoir atteint la prostate P alors qu'ils la comprimaient déjà.

Selon l'invention, les moyens d'analyse 11 comportent ainsi des moyens d'estimation 12 d'une distance entre la sonde 5 et la paroi rectale qu'il convient de percer pour atteindre la prostate P. Lesdits moyens d'estimation 12 estiment cette distance en temps réel par analyse des images de la sonde 5. L'organe de commande 10 génère alors des ordres de commande pour opposer une résistance au déplacement du support 1 en fonction de ladite distance de façon que le support 1 soit de plus en plus freiné au fur et à mesure que l'aiguille 2 s'approche de la prostate P.

Le praticien manipulant le dispositif de l'invention conjointement au bras articulé 9 ressent une résistance de plus en plus importante de la part du bras articulé 9 et peut ainsi estimer plus facilement à quel distance il se trouve de la prostate P. Un retour d'effort est donc artificiellement réalisé. De façon avantageuse, le retour d'effort est réalisé sans capteur d'effort.

Selon l'invention, l'organe de commande 10 ordonne un freinage du bras articulé 9 comme suit.

Lors d'une séquence d'initialisation, au cours d'une première étape, les moyens d'acquisition acquièrent une première image dite image de référencer.

Ladite image de référence est ensuite transmise à l'organe de commande 10. L'image de référence est par exemple une image générale de la prostate P ou bien une image d'une zone particulière de la prostate P. L'image de référence est par exemple en deux dimensions ou en trois dimensions. En variante, l'image de référence n'est pas acquise par les moyens d'acquisition mais est acquise par d'autres analyses médicales (comme une Imagerie par Résonnance Magnétique) et fournie à l'organe de commande 10.

Lors d'une séquence de déplacement de l'instrument médical, les moyens d'acquisition acquièrent au moins une image et la transmettent à l'organe de commande 10.

Les moyens d'estimation calculent alors par comparaison entre l'image et l'image de référence l'axe de déformation principale (soit la direction de déformation principale) de la prostate P ainsi que la valeur de la déformation de la prostate P selon cet axe.

Puis, les moyens d'estimation transposent la direction de déformation principale et la valeur de cette déformation principale dans un repère lié au bras articulé 9, la position de la sonde 5 relativement au bras articulé 9 étant connue comme il a déjà été vu.

Ensuite, les moyens d'estimation déterminent la distance entre l'instrument médical 2 (et donc la sonde 5) relativement à la prostate P à partir de l'indication de la direction et de la valeur de la déformation principale dans le repère du bras articulé 9.

L'organe de commande 10 gère alors le bras articulé 9 de façon à ce que les mouvements de l'instrument médical 2 et de la sonde 5 soient freinés proportionnellement à la distance entre l'instrument médical 2 et la prostate P.

Les moyens d'analyse comportent des moyens d'estimation 13 d'une déformée de la prostate P lorsque la sonde 5 est déjà en contact avec la prostate au travers de la paroi rectale. Les moyens d'estimation d'une déformée 13 estiment cette déformée en temps réel par analyse des images de la sonde 5. Par exemple, les moyens d'estimation 13 d'une déformée comportent des moyens d'estimation 15 d'une distance d'écrasement de la prostate P par la sonde 5. L'organe de commande 10 génère alors des ordres de commande pour opposer une résistance au déplacement du bras articulé 9 en fonction de ladite déformée de façon que le bras articulé 9 soit de plus en plus freiné au fur et à mesure que l'aiguille 2 s'enfonce dans la prostate P et donc déforme la prostate. On crée ainsi une raideur artificielle aidant le praticien dans le maintien de ses gestes. En particulier, on évite ainsi que le praticien compresse trop la prostate P.

Il est par exemple possible de commander le bras articulé par rapport à la déformée de la prostate P par exemple en estimant les efforts générés par le bras articulé sur la prostate P par toute formule logique (par exemple relation de proportionnalité, avec ou sans amortissement, reproduction de la loi de comportement de l'organe...)...

Selon un mode de réalisation particulier, à partir de la déformée de la prostate P, que l'instrument soit ou non au contact de la prostate P, il est possible d'estimer la distance entre une zone particulière à atteindre sur la prostate P (par exemple pour réaliser une ponction au niveau de cette zone) et l'instrument médical 2. En effet, si une zone particulière de la prostate (appelée cible) est définie et entrée dans l'organe de commande 10, l'organe de commande 10 calcule à partir d'une image acquise par les moyens d'acquisition la direction selon laquelle l'instrument médical 2 doit se déplacer pour atteindre ladite cible ainsi que la distance séparant l'instrument médical 2 et ladite cible selon cette direction. L'instrument médical 2, et donc la sonde 5, ayant une position connue par rapport au bras articulé 9, la distance et la direction précitées peuvent être exprimées dans un repère lié au bras articulé 9.

L'organe de commande 10 peut dès lors générer des ordres de commande à destination du bras articulé afin par exemple que le bras articulé 9 oppose une résistance si le praticien tend à éloigner l'instrument médical 2 de la cible ou au contraire fin que le bras articulé 9 entraîne le mouvement du praticien dans la bonne direction.

Selon un mode de réalisation privilégié, l'organe de commande 10 comporte des moyens de sélection 14 d'un mode de manipulation du bras articulé 9. Selon un premier mode de manipulation du bras articulé 9, le bras articulé 9 réalise seul le déplacement du support 1. Selon un deuxième mode de manipulation du bras articulé 9, le praticien déplace également le support 1 conjointement au bras articulé 9, par exemple au niveau de l'extrémité proximale la du support 1. A cet effet, l'extrémité proximale la est de préférence conformée en une poignée. Le bras articulé 9 est alors co-manipulable. Selon un troisième mode de manipulation du bras articulé 9, le praticien ne peut déplacer le support 1 que pour certains degrés de liberté. Le bras articulé 9 est alors partiellement co-manipulable.

Le praticien peut ainsi choisir jusqu'à quel point il souhaite être assisté par le bras articulé 9. Le dispositif de l'invention s'adapte ainsi aux besoins du praticien en étant possiblement co-manipulable.

Le dispositif selon l'invention permet d'aider le praticien à positionner avec précision l'aiguille 2 vis-à-vis de la prostate P et également d'améliorer une sensation tactile du praticien.

Le bras articulé 9 n'est donc pas le seul à agir sur la position de l'aiguille 2 : le praticien manipule également le support 1 et donc indirectement l'aiguille 2. Ainsi, c'est la combinaison des efforts exercées sur le support par le praticien d'une part et par le bras articulé 9 d'autre part qui va déterminer les mouvements et la position de l'aiguille.

Ainsi le support 1 est manipulé à la fois par le praticien et par le bras articulé 9, ce qui permet d'agir sur la position de l'aiguille et sur le ressenti tactile du praticien.

En effet, la génération d'efforts par le bras articulé 9 n'a pas pour but de manipuler seul l'aiguille, mais de modifier le ressenti du praticien et donc ses gestes, notamment par exemple en lui permettant de ressentir des efforts représentatifs de l'interaction entre l'aiguille 2 et la prostate P ou en modifiant son geste de façon à positionner l'aiguille dans la position cible.

Selon un mode de réalisation privilégié, les moyens de déplacement sont liés à l'extrémité proximale la du support 1 par une liaison de type à rotule R.

Il se peut que le patient ne soit pas sous anesthésie locale et/ou qu'il ne soit pas immobilisé. Ainsi, l'anus dans lequel est introduit l'aiguille 2 n'est pas nécessairement fixe. De façon avantageuse, la liaison rotule R évite que les moyens de déplacement ne s'opposent à des mouvements naturels du patient. Le dispositif de l'invention respecte donc l'anatomie du patient.

Le dispositif de l'invention comporte un écran de visualisation 7 des images prises par la sonde 5. L'écran de visualisation est relié à l'organe de commande 10. Ainsi, un praticien peut avoir un retour visuel et estimer la distance de l'aiguille 2 relativement à la prostate P.

De préférence, le dispositif de l'invention comporte des moyens de blocage du support 1. Ainsi, une fois que l'aiguille 2 est correctement positionnée vis-à-vis de la prostate P, le praticien peut bloquer un déplacement du support 1 et du bras articulé 9 avant d'insérer l'aiguille 2 dans la prostate P.

Bien entendu l'invention n'est pas limitée au mode de réalisation décrit et on peut y apporter des variantes de réalisation sans sortir du cadre de l'invention tel que défini par les revendications.

Le dispositif pourra approcher un tout autre instrument médical près d'un organe interne qu'un porte-aiguille portant une aiguille. De plus, le dispositif pourra approcher un instrument médical de tout autre organe qu'une prostate. En particulier, le dispositif n'est pas exclusivement destiné à un appareil génital masculin. En outre, bien qu'ici on atteigne l'organe voulu en introduisant le support par une voie naturelle qu'est le rectum, on pourra atteindre l'organe voulu en introduisant le support dans le corps du patient par une voie artificielle comme un trocart.

La sonde pourra être d'un autre type qu'échographique, par exemple un dispositif optique ou infrarouge.

Les moyens de déplacement pourront être reliés à l'extrémité proximale du support par une autre liaison qu'une liaison rotule comme par exemple une liaison de type à cardan.

L'organe de commande pourra comporter des moyens de sélection d'un mode de manipulation des moyens de déplacement quels qu'ils soient. En particulier l'organe de commande pourra comporter des moyens de sélection d'un mode de manipulation des moyens de déplacement dans lequel les moyens de déplacement sont co-manipulables. L'organe de commande pourra comporter des moyens de sélection d'un mode de manipulation des moyens de déplacement dans lequel les moyens de déplacement sont partiellement co-manipulable.

Le bras articulé pourra être conformé pour permettre de déplacer l'extrémité proximale du support selon un nombre quelconque de degrés de liberté à condition qu'il puisse au moins permettre un déplacement de l'extrémité proximale du support à l'intérieur du corps du patient.

## Revendications

1. Dispositif d'assistance au positionnement d'un instrument médical (2) inséré dans une voie naturelle (100) ou une voie artificielle d'un patient relativement à un organe interne (P) d'un patient, le dispositif comportant :
- un support (1) destiné à être inséré au moins en partie dans le corps du patient et qui porte l'instrument médical ;
- des moyens de déplacement du support, lesdits moyens de déplacement comportant un bras articulé (9) qui comprend une pluralité de degrés de liberté pour déplacer une extrémité proximale (1a) du support ;
- des moyens d'acquisition d'images de l'organe interne pour positionner l'instrument médical relativement à l'organe interne, les moyens d'acquisition d'images comportant une sonde (5) portée par le support de sorte que l'instrument médical et la sonde soient rigidement liés ;
- un organe de commande (10) des moyens de déplacement qui est relié aux moyens d'acquisition d'images et qui comporte des moyens d'analyse des images (11) pour générer des ordres de commande à destination du bras articulé afin de contrôler au moins un déplacement de l'instrument médical relativement à l'organe interne, les moyens d'analyse comportant des moyens d'estimation d'une distance (12) entre la sonde (5) et l'organe interne pour freiner un déplacement du bras articulé (9) en fonction de ladite distance, de façon que le bras articulé (9) soit de plus en plus freiné au fur et à mesure que l'instrument s'approche de l'organe, les moyens d'analyse comportant des moyens d'estimation d'une déformée (13) de l'organe interne pour freiner un déplacement du bras articulé (9) en fonction de ladite déformée, de façon que le bras articulé (9) soit de plus en plus freiné au fur et à mesure que l'instrument (2) s'enfonce dans l'organe.

2. Dispositif selon la revendication 1, dans lequel le bras articulé (9) est lié à l'extrémité proximale (1a) du support (3) par une liaison de type à cardan ou à rotule.

3. Dispositif selon la revendication 1, dans lequel la sonde (5) est une sonde échographique.

4. Dispositif selon la revendication 1, comportant un écran de visualisation (7) des images prises par la sonde (5).

5. Dispositif selon la revendication 1, dans lequel l'instrument médical est une aiguille (2).

## Patentansprüche

1. Vorrichtung zur Unterstützung der Positionierung eines medizinischen Instruments (2), das in einen natürlichen Weg (100) oder einen künstlichen Weg eines Patienten eingefügt ist, relativ zu einem inneren Organ (P) eines Patienten, wobei die Vorrichtung umfasst:
- einen Träger (1), der dazu bestimmt ist, zumindest teilweise in den Körper des Patienten eingefügt zu werden, und der das medizinische Instrument trägt;
- Verschiebungsmittel zum Verschieben des Trägers, wobei die genannten Verschiebungsmittel einen angelenkten Arm (9) umfassen, der eine Vielzahl von Freiheitsgraden hat, um ein proximales Ende (1a) des Trägers zu verschieben,
- Bilderfassungsmittel zum Erfassen von Bildern des inneren Organs, um das medizinische Instrument relativ zu dem inneren Organ zu positionieren, wobei die Bilderfassungsmittel eine Sonde (5) umfassen, die von dem Träger derart getragen wird, dass das medizinische Instrument und die Sonde starr verbunden sind;
- ein Steuerelement (10) zum Steuern der Verschiebungsmittel, das mit den Bilderfassungsmitteln verbunden ist und das Bildanalysemittel (11) umfasst, um Steuerbefehle für den angelenkten Arm zu erzeugen, um zumindest eine Verschiebung des medizinischen Instruments relativ zum inneren Organ zu steuern, wobei die Analysemittel Schätzmittel (12) zum Schätzen eines Abstandes zwischen der Sonde (5) und dem inneren Organ umfassen, um eine Verschiebung des angelenkten Arms (9) in Abhängigkeit von dem genannten Abstand zu bremsen, derart, dass der angelenkte Arm (9) zunehmend gebremst wird, in dem Maße wie sich das Instrument (2) an das Organ annähert,
wobei die Analysemittel Schätzmittel (13) zum Schätzen einer Verformung des inneren Organs umfassen, um eine Verschiebung des angelenkten Arms (9) in Abhängigkeit von der genannten Verformung zu bremsen, derart, dass der angelenkte Arm (9) zunehmend gebremst wird, in dem Maße wie das Instrument (2) in das Organ eindringt.

2. Vorrichtung nach Anspruch 1, bei der der angelenkte Arm (9) mit einem proximalen Ende (1a) des Trägers (3) über eine Verbindung der Art Kardangelenk oder Kugelgelenk verbunden ist.

3. Vorrichtung nach Anspruch 1, bei der die Sonde (5) eine echographische Sonde ist.

4. Vorrichtung nach Anspruch 1, umfassend einen Anzeigebildschirm (7) zum Anzeigen der von der Sonde (5) aufgenommenen Bilder.

5. Vorrichtung nach Anspruch 1, bei der das medizinische Instrument eine Nadel (2) ist.

## Claims

1. An assistive device for positioning a medical instrument (2), inserted into a natural duct (100) or an artificial duct of a patient, relative to an internal organ (P) of a patient, the device having:
- a support (1) which is dedicated to be inserted at least in part into the body of the patient and which supports the medical instrument;
- movement means for moving the support, said movement means having an articulated arm (9) which has a plurality of degrees of freedom for moving a proximal end (1a) of the support;
- image acquisition means for acquiring images of the internal organ in order to position the medical instrument relative to the internal organ, the image acquisition means having a probe (5) carried by the support, such that the medical instrument and the probe are rigidly connected;
- a control unit (10) which controls the movement means, is connected to the image acquisition means and has image analysis means (11) for generating control commands for the articulated arm in order to control at least one movement of the medical instrument relative to the internal organ, the analysis means having means (12) for estimating a distance between the probe (5) and the internal organ in order to brake a movement of the articulated arm (9) as a function of said distance, so that the articulated arm (9) is braked more and more as the instrument approaches the organ, in which the analysis means have means (13) for estimating a deformation of the internal organ in order to brake a movement of the articulated arm (9) as a function of said deformation so that the articulated arm (9) is braked more and more as the instrument (2) engages in the organ.

2. The device as claimed in claim 1, in which the articulated arm (9) is connected to the proximal end (1a) of the support (3) by a connection of the cardan or ball-joint type.

3. The device as claimed in claim 1, in which the probe (5) is an ultrasound probe.

4. The device as claimed in claim 1, having a display screen (7) for the images taken by the probe (5) .

5. The device as claimed in claim 1, in which the medical instrument is a needle (2).
